# EUROPÄISCHE PATENTANMELDUNG

(11) **EP 4 202 467 A1**
(43) Veröffentlichungstag der Anmeldung: **28.06.2023**
(21) Anmeldenummer: 21217857.8
(22) Anmeldetag: 27.12.2021
(51) Int. Cl.: G01R 33/28, G01R 33/54, A61B 34/20

(54) **VORRICHTUNG UND VERFAHREN ZUR FÜHRUNG EINES INSTRUMENTS MITTELS EINES LEUCHTZEIGERS**

(71) Anmelder: Siemens Healthcare GmbH, 91052 Erlangen (DE)
(72) Erfinder: Hengerer, Arne, 91096 Möhrendorf (DE)

(57) **Zusammenfassung**

Die Erfindung betrifft ein Instrument, einen Magnetresonanztomographen sowie ein Verfahren zum Betrieb des Magnetresonanztomographen mit dem Instrument, bei dem das Instrument an einem Patienten ausgerichtet wird. Das medizinische Instrument weist einen ersten Projektor zur Projektion eines Leuchtzeigers in einer vorbestimmten Ausrichtung relativ zu dem medizinischen Instrument auf. Der Magnetresonanztomograph weist eine Positionierhilfe auf, die ausgelegt ist, eine vorbestimmte Position an einer Innenwand eines Patiententunnels für einen Anwender sichtbar auszuzeichnen. In dem Verfahren wird eine vorbestimmten Ausrichtung des ersten Projektors relativ zu dem medizinischen Instrument oder Position der Positionierhilfe an der Wand des Patiententunnels ermittelt, in der der Leuchtzeiger des ersten Projektors in Deckung mit der Positionierhilfe kommt, wenn das medizinische Instrument parallel zu einer Trajektorie durch einen Eintrittspunkt am Patienten und einem Zielpunkt im Patienten ausgerichtet ist und das medizinische Instrument so ausgerichtet, dass der Leuchtzeiger und die Positionierhilfe in Deckung sind.

## Beschreibung

Die Erfindung betrifft ein Instrument, einen Magnetresonanztomographen sowie ein Verfahren zum Betrieb des Magnetresonanztomographen mit dem Instrument, bei dem das Instrument an einem Patienten ausgerichtet wird.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden.

Auf vorteilhafte Weise erlaubt ein Magnetresonanztomograph eine Visualisierung des Körperinneren über längere Zeit, ohne den Patienten oder Operateur einer erhöhten Dosis ionisierender Strahlung auszusetzen. Es sind jedoch Instrumente aus Kunststoff oder Metall durch die Magnetresonanztomographie nicht oder nur indirekt zu erfassen und damit eine Ausrichtung nur schwierig einzustellen.

Es ist eine Aufgabe der Erfindung, ein Verfahren und eine Vorrichtung bereitzustellen, die mit geringem zusätzlichem Aufwand ein Positionieren und ein Ausrichten eines Instruments erlauben.

Die Aufgabe wird durch ein erfindungsgemäßes Instrument nach Anspruch 1, einen erfindungsgemäßen Magnetresonanztomographen nach Anspruch 5, einem erfindungsgemäßen System nach Anspruch 8 und ein erfindungsgemäßes Verfahren nach Anspruch 9 gelöst.

Das erfindungsgemäße medizinisches Instrument ist zur Anwendung an oder in einem Patienten vorgesehen. Das Instrument kann beispielsweise eine Biopsienadel sein, ein Instrument zur Ablation oder zum Applizieren einer Brachytherapie.

Das erfindungsgemäße medizinische Instrument weist einen ersten Projektor zur Projektion eines Leuchtzeigers in einer vorbestimmten Ausrichtung relativ zu dem medizinischen Instrument auf. Als Leuchtzeiger wird ein von dem Projektor auf einer Innenfläche eines Patiententunnels erzeugtes Lichtmuster angesehen, das eine Ausrichtung des Projektors und damit des Instruments mit einem Winkelfehler kleiner als 5 Grad, 2 Grad oder 1 Grad erlaubt.

Der Projektor kann entsprechend den nachfolgend beschriebenen Ausführungsformen beispielsweise eine einfache Lichtquelle mit einer Optik zur Ausrichtung in einen parallelen Strahl sein, ein Laser mit einer Kollimationsoptik, aber auch ein Projektor, der ein Muster mit einer Lichtquelle durchleuchtet und auf die Innenwand projiziert. Denkbar wäre auch eine veränderliche Projektion durch eine Technologie vergleichbar einem Beamer oder durch Projektion einer OLED-Matrix mit einer Optik.

Bei dem erfindungsgemäßen Instrument ist die vorbestimmte Ausrichtung des ersten Projektors bei einer anwendungsgemäßen Positionierung des medizinischen Instruments am Patienten von dem Patienten weg. Mit anderen Worten, der Leuchtzeiger des Projektors trifft bei einer anwendungsgemäßen Anordnung des medizinischen Instruments nicht auf den Patienten, sondern auf die umgebende Innenwand des Patiententunnels.

Auf vorteilhafte Weise erlaubt der Projektor einen zweiten Referenzpunkt für die Ausrichtung des medizinischen Instruments auf der Wand des Patiententunnels zu erzeugen und zusätzlich zu dem Aufsetzpunkt bzw. Eintrittspunkt am Patienten zu nutzen.

Der erfindungsgemäße Magnetresonanztomograph weist eine Positionierhilfe auf. Die Positionierhilfe ist ausgelegt, eine vorbestimmte Position an einer Innenwand eines Patiententunnels für einen Anwender sichtbar auszuzeichnen. Mit anderen Worten, die Positionierhilfe erlaubt es einem Nutzer, einen Ort an der Innenwand des Tunnels zu identifizieren, beispielsweise als Zielmarke für den Lichtzeiger des ersten Projektors des erfindungsgemäßen medizinischen Instruments. Die Positionierhilfe kann beispielsweise eine einzelne im Reflexionsvermögen zur umgebenden Innenwand unterschiedliche Markierung sein. Denkbar ist auch eine Erhebung oder Vertiefung an der Oberfläche der Innenwand. Denkbar sind aber auch temporäre Markierungen, die von dem Magnetresonanztomographen veränderlich sichtbar gemacht werden können. Näheres ist nachfolgend zu den Unteransprüchen ausgeführt.

Auf vorteilhafte Weise erlauben die Positionierhilfen in Wechselwirkung mit dem erfindungsgemäßen Instrument eine definierte Ausrichtung des Instruments.

Das erfindungsgemäße Verfahren betrifft die Positionierung eines bereits beschriebenen medizinischen Instruments mit einem ersten Projektor zum Projizieren eines Leuchtzeigers und einem erfindungsgemäßen Magnetresonanztomographen mit einer Positionierhilfe. Das Verfahren wird weiterhin mit einer Planungssteuerung mit einer Bedienoberfläche zur Planung eines Eingriffs mit dem medizinischen Instrument ausgeführt. Die Planungssteuerung kann dabei als Programm auf dem Magnetresonanztomographen ausgeführt sein und dessen Ausgabemittel nutzen, aber auch auf einem anderen Computer oder in einer Cloud als separate Einheit.

In einem Schritt des erfindungsgemäßen Verfahrens wird der Patient in dem Magnetresonanztomographen positioniert. Dies erfolgt so, dass ein vorbestimmter Eintrittspunkt für das medizinische Instrument an der Oberfläche des Patienten und ein vorbestimmter Zielpunkt des Eingriffs in dem Patienten sich in einem Erfassungsbereich des Magnetresonanztomographen befinden. Der Erfassungsbereich wird auch als Field of View (FoV) bezeichnet. Das Positionieren erfolgt vorzugsweise mittels der Patientenliege. Der Eintrittspunkt kann beispielsweise mit einem Magnetresonanz-aktiven Marker gekennzeichnet sein.

In einem weiteren Schritt des erfindungsgemäßen Verfahrens wird eine vorbestimmte Ausrichtung des ersten Projektors relativ zu dem medizinischen Instrument und/oder Position der Positionierhilfe an der Wand des Patiententunnels ermittelt, in der der Leuchtzeiger des ersten Projektors in Deckung mit der Positionierhilfe kommt, wenn das medizinische Instrument parallel zu einer Trajektorie durch den Eintrittspunkt und den Zielpunkt ausgerichtet ist. Es ist die Idee der Erfindung, die Ausrichtung des Instruments zu erreichen, indem wie in dem nachfolgenden Schritt ausgeführt, ein Benutzer den Leuchtzeiger auf die Positionierhilfe ausrichtet. Dabei ist es denkbar, dass entweder die Position der mit dem Leuchtzeiger anzuvisierenden Positionierungshilfe verändert wird, um eine veränderte Ausrichtung zu unterstützen bzw. zu determinieren, oder die Ausrichtung des ersten Projektors relativ zu dem medizinischen Instrument. Entsprechend wird in diesem Schritt die Position der Positionierhilfe oder die Ausrichtung des ersten Projektors relativ zu dem Instrument bestimmt, unter der das medizinische Instrument parallel zu der Trajektorie ausgerichtet ist, wenn der Leuchtzeiger die Positionierhilfe trifft. Die Berechnung erfolgt gemäß der analytischen Geometrie in der Steuerung des Magnetresonanztomographen, der Planungssteuerung oder einem anderen Rechner unter Anwendung von Drehungen und Bestimmen eines Schnittpunktes einer Geraden mit einer Oberfläche des Patientenunnels. Denkbar ist grundsätzlich auch, dass beide Parameter, Position der Positionierhilfe und Ausrichtung des ersten Projektors gleichzeitig verändert werden und demgemäß ermittelt werden.

In einem anderen Schritt des erfindungsgemäßen Verfahrens wird der erste Projektor in die ermittelte relative Ausrichtung zu dem medizinischen Instrument gebracht. Dies kann beispielsweise durch Aktoren an dem Projektor erfolgen, die über eine vorzugsweise drahtlose Signalverbindung mit der Steuerung des Magnetresonanztomographen stehen.

Ergänzend oder alternativ wird die Positionierhilfe ausgegeben. Im einfachsten Fall kann dies die Ausgabe von Koordinaten für den Nutzer auf einer Ausgabe sein. Die Koordinaten beziehen sich dabei auf ein Koordinatensystem, das auf der Innenseite des Patiententunnels sichtbar angebracht ist und für den Nutzer anhand der Koordinaten ein eindeutige Zielposition für den Leuchtzeiger als Positionierhilfe angibt. Denkbar ist aber auch eine Positionierhilfe, die beispielsweise ein Gitter aus Lichtpunkten angibt, von denen der der zu erzielende Position des Leuchtzeigers am nächsten liegende Lichtpunkt aktiviert wird. Für eine bessere Kompatibilität mit dem Magnetresonanztomographen können die Lichtpunkte auch mittels Glasfaser realisiert werden, bei der die Lichtquelle entfernt vom Patiententunnel angeordnet ist. Möglich wäre auch eine Projektion der Positionierhilfe. Der Leuchtzeiger wird aktiviert, sofern er nicht bereits aktiv ist.

Auf vorteilhafte Weise erlaubt das erfindungsgemäße Verfahren, dem Nutzer eine einfache und zuverlässige Hilfe für die Ausrichtung des medizinischen Instruments bereitzustellen.

Weitere vorteilhafte Ausführungsformen sind zu den Unteransprüchen angegeben.

In einer denkbaren Ausführungsform des erfindungsgemäßen medizinischen Instruments weist das medizinische Instrument eine Symmetrieachse auf. Die Symmetrieachse kann eine Spiegelsymmetrieachse sein, vorzugsweise handelt es sich aber um eine Achse für eine Rotationssymmetrie.

Auf vorteilhafte Weise erlaubt es eine Ausrichtung des ersten Projektors parallel zu einer Symmetrieachse, insbesondere einer Rotationsachse, das medizinische Instrument in unterschiedlichen Drehpositionen zu halten, ohne dadurch die Ausrichtung zu verändern. Insbesondere bei rotationssymmetrischen Instrumenten wie einer Biopsienadel wird dadurch die Ausrichtung vereinfacht.

In einer möglichen Ausführungsform des medizinischen Instruments weist das Instrument einen Steuereingang auf und ist ausgelegt, die vorbestimmte Ausrichtung des ersten Projektors in Abhängigkeit von einer Anweisung über den Steuereingang auf vorbestimmte Weise zu ändern. Unter Ausrichtung des Projektors ist dabei die Ausrichtung der Projektion des Leuchtzeigers zu dem medizinischen Instrument zu verstehen. Es dabei denkbar, dass der erste Projektor durch Aktuatoren mechanisch relativ zu dem Instrument aufgrund der Anweisung ausgerichtet wird. Es kann aber der Projektor auch dazu ausgelegt sein, die Ausrichtung des Leuchtzeigers zu verändern, beispielsweise indem der Projektor darauf beruht, dass ein Bild mit dem Leuchtzeiger projiziert wird und dieses Bild verändert wird, indem die Position des Leuchtzeigers innerhalb des Bildes verändert wird. Dies kann beispielsweise durch die Projektion einer OLED-, DLP- oder LCD-Matrix erreicht werden. Der Steuereingang kann beispielsweise eine elektrische oder optische Signalleitung sein oder auch eine drahtlose Datenübertragung, beispielsweise mittels Bluetooth oder WLAN.

Auf vorteilhafte kann bei einer veränderlichen Ausrichtung des ersten Projektors eine einzelne Positionierhilfe oder zumindest eine geringe Zahl an Positionierhilfen an der Innenwand des Patiententunnels genügen, um eine präzise Ausrichtung zu gewährleisten.

In einer denkbaren Ausführungsform des medizinischen Instruments weist das Instrument einen Sensor auf, der ausgelegt ist, eine Ausrichtung des Instruments um die Symmetrieachse relativ zu dem Magnetresonanztomographen zu erfassen. Denkbar wäre z.B. ein Sensor, der die Richtung der Schwerkraft ermittelt oder des B0-Magnetfeldes.

Wenn der erste Projektor relativ zu dem Instrument ausgerichtet wird, kann dieses vom Nutzer dennoch verdreht werden. Wird die Drehung um die Symmetrieachse mittels des Sensors bestimmt, so kann auf vorteilhafte Weise die Ausrichtung des ersten Projektors eingestellt werden, um unter Berücksichtigung der der Drehung den Leuchtzeiger korrekt auszurichten.

Es ist aber auch denkbar, dass der Leuchtzeiger und die Positionierhilfe ausgelegt sind, beispielsweise durch ein Muster und/oder eine Form des Leuchtzeigers, eine Rotation des Instruments um die Symmetrieachse zu identifizieren. In Verbindung mit einer Positionsmarkierung kann so gleichzeitig die Ausrichtung der Trajektorie und Rotation um die Symmetrieachse angezeigt und damit der Nutzer die Ausrichtung korrekt eingestellt werden. Beispielsweise können Leuchtzeiger und Positionierhilfe einen gerichteten Pfeil aufweisen mit einem Punkt oder Kreis am Ursprung. Wenn der Nutzer die Ursprungsmarkierung und den Pfeil in Deckung bringt, ist dadurch die Ausrichtung des Instruments auch bei einer Abweichung der Rotationsachse des Instruments von der Projektionsrichtung des ersten Projektors möglich.

In einer möglichen Ausführungsform des erfindungsgemäßen Magnetresonanztomographen weist die Positionierhilfe ein sichtbar auf der Innenseite des Patiententunnels angebrachte Markierung auf. Denkbar sind farblich oder im Reflexionsverhalten abgesetzte Markierungen oder in der Form von der Oberfläche abweichende Vertiefungen oder Erhebungen als Positionierhilfe. Die Positionierhilfe kann auch ein Koordinatensystem auf der Innenwand des Patiententunnels aufweisen.

Auf vorteilhafte Weise ist eine sichtbare Positionierhilfe einfach anzubringen und anzuwenden.

In einer anderen möglichen Ausführungsform des erfindungsgemäßen Magnetresonanztomographen weist der Magnetresonanztomograph einen zweiten Projektor auf. Der zweite Projektor ist ausgelegt, eine vorbestimmte Position an der Innenwand des Patiententunnels mittels einer Lichtprojektion als Positionierhilfe sichtbar auszuzeichnen. Wie zuvor zum ersten Projektor beschrieben, kann der zweite Projektor ein Laser oder Pointer sein, der einen einzelnen Lichtpunkt an die Innenwand wirft, oder auch ein Symbol oder ein ganzes Muster wie ein Koordinatensystem. Insbesondere ist es auch denkbar, dass der zweite Projektor geeignet ist, unterschiedliche Positionen auszuzeichnen, beispielsweise indem er ein Muster an unterschiedlichen Positionen projiziert. Dies kann beispielsweise erreicht werden, indem die Ausrichtung des zweiten Projektors von der Planungssteuerung veränderbar ist oder unterschiedliche Bilder projizierbar sind, mit dem Symbol bzw. Muster an unterschiedlichen Positionen.

Auf vorteilhafte Weise erlaubt der zweite Projektor die Auszeichnung veränderlicher Positionen an der Innenwand des Patiententunnels, um mit einem medizinischen Instrument unterschiedliche Ausrichtungen zu erzielen.

In einer denkbaren Ausführungsform des erfindungsgemäßen Verfahrens weist das Verfahren weiterhin den Schritt auf, das medizinische Instrument derart auszurichten, dass der Leuchtzeiger mit der Positionierhilfe in Deckung gelangt. Der Nutzer des medizinischen Instruments setzt dazu das medizinische Instrument mit dem Ende oder der Spitze, die der Richtung der Projektion des ersten Projektors entgegengesetzt ist, an dem vorbestimmten Eintrittspunkt auf. Der Eintrittspunkt kann vorzugsweise mit einem MR-aktiven Marker markiert sein. Der Nutzer neigt und schwenkt anschließend das medizinische Instrument derart, dass der Leuchtzeiger und die Positionierhilfe in Deckung gelangen. Sind der Leuchtzeiger und die Positionierhilfe nicht rotationssymmetrisch ausgelegt, sondern haben eine Vorzugsrichtung, um eine Drehung des Instruments um die Trajektorie anzugeben, so werden der Leuchtzeiger und die Positionierhilfe wie vorbestimmt zueinander ausgerichtet, um die gewünschte Orientierung zu erzielen. Beispielsweise können Zeiger, wie schon beschrieben, in Deckung gebracht werden.

Das Ausrichten mittels des Leuchtzeigers stellt auf einfache Weise die gewünschte Ausrichtung des medizinischen Instruments auf den Zielpunkt sicher.

In einer möglichen Ausführungsform des erfindungsgemäßen Verfahrens weist das Verfahren weiterhin die Schritte auf, eine Abbildung eines Patienten auf der Bedienoberfläche der Planungssteuerung bereitzustellen, und mittels der Abbildung einen Eintrittspunkt auf der Patientenoberfläche und einen Zielpunkt in dem Patienten durch den Nutzer festzulegen. Denkbar ist beispielsweise, dass der Eintrittspunkt in der Abbildung mittels eines MR-aktiven Markers sichtbar ist oder durch eine Verformung der Oberfläche durch das Instrument oder ein anderes Objekt, wie z.B. einen Finger des Nutzers, sichtbar wird. Der Zielpunkt kann durch den Nutzer in der Abbildung markiert werden, beispielsweise erkenntlich anhand eines höheren Kontrasts aufgrund höherer Dichte in einem bestimmten Bereich. Möglich wäre auch eine automatische Segmentierung des Zielpunktes durch die Planungssteuerung.

Auf vorteilhafte Weise kann durch die Auswahl an einer Abbildung die Trajektorie für das Verfahren einfach bestimmt werden.

In einer denkbaren Ausführungsform des erfindungsgemäßen Verfahrens ist die Ausrichtung des ersten Projektors zu dem medizinischen Instrument fest und vorzugsweise (anti-)parallel zu einer Trajektorie des medizinischen Instruments.

In dem Schritt Ermitteln der Ausrichtung wird dann lediglich eine Position der Positionierhilfe an der Wand des Patiententunnels ermittelt, in der der Leuchtzeiger des ersten Projektors in Deckung mit der Positionierhilfe kommt, wenn das medizinische Instrument parallel zu einer Trajektorie durch den Eintrittspunkt und den Zielpunkt ausgerichtet ist. Dies wird erreicht, indem ein Schnittpunkt des Leuchtzeigers mit der Innenseite des ermittelt wird, z.B. durch Lösen eines Gleichungssystems mit einer Geraden kongruent zu der Trajektorie und einer mathematischen Beschreibung der Innenwand des Patiententunnels durch einen Zylinder in der Planungssteuerung ermittelt wird. Anschließend wird der Schnittpunkt ausgegeben.

Auf vorteilhafte Weise ist das Bestimmen des Schnittpunktes besonders einfach, wenn die Trajektorie parallel zu der Projektionsrichtung des Leuchtzeigers ist

In einer möglichen Ausführungsform des erfindungsgemäßen Verfahrens wird der Schnittpunkt als Koordinate an einer Bedienschnittstelle dem Nutzer ausgegeben.

Dabei ist vorzugsweise auf der Innenwand des Patiententunnels ein sichtbares Koordinatensystem angeordnet. Das Koordinatensystem erlaubt dem Nutzer eine eindeutige Identifizierung einer Position auf Innenwand mittels der Koordinaten, die zur Identifikation des Schnittpunkts ausgegeben werden. Der Nutzer kann dann diese durch die Koordinaten identifizierte Position als Positionierhilfe mit dem Leuchtsucher anvisieren und so die gewünschte Ausrichtung des medizinischen Instruments erzielen.

In einer denkbaren Ausführungsform des erfindungsgemäßen Verfahrens ist das Ausgeben des Schnittpunktes an der Innenseite des Patiententunnels ein Markieren mittels des zweiten Projektors. Denkbar ist es beispielsweise, dass der zweite Projektor durch ein Stellglied auf Anweisung der Planungssteuerung so ausgerichtet wird, dass eine Projektion des zweiten Projektors als Positionierhilfe die ermittelten Koordinaten des Schnittpunktes auf der Innenwand des Patiententunnels auszeichnet. Denkbar ist auch, dass ein von dem zweiten Projektor an die Innenwand projizierte Bild so verändert wird, dass es die ermittelte Koordinate auszeichnet. Es könnte auch ein Raster an Lichtunkten mittels LED oder Glasfaser an der Innenwand vorgesehen sein, wobei der dem ermittelten Schnittpunkt am nächsten Liegenden Lichtpunkt von der Planungssteuerung als Positionierhilfe aktiviert wird.

Auf vorteilhafte Weise vereinfacht das aktive Markieren des veränderlichen Schnittpunktes dem Nutzer das Auffinden des Zielpunktes für den Leuchtzeiger und damit das Ausrichten des Instruments.

In einer anderen Ausführungsform des erfindungsgemäßen Verfahrens ist die Ausrichtung des ersten Projektors relativ zu dem Instrument einstellbar. In dem Schritt ermitteln der Ausrichtung wird dabei eine Ausrichtung des ersten Projektors relativ zu dem medizinischen Gerät bestimmt, unter der der Lichtzeiger bei Ausrichtung des medizinischen Instruments parallel zu einer Trajektorie durch den Eintrittspunkt und den Zielpunkt auf die Positionierhilfe ausgerichtet ist. Da dabei ist die Ausrichtung des ersten Projektors nicht mehr parallel zu der Trajektorie und damit abhängig von einem Winkel einer Rotation des Instruments um die Trajektorie. Es ist dabei denkbar, dass ein Sensor wie beispielsweise ein Magnetfeldsensor oder ein Schwerkraftsensor in dem Instrument den Winkel erfassen und die Ausrichtung des ersten Projektors davon abhängig einstellen.

Denkbar ist aber auch, dass der erste Leuchtzeiger nicht rotationssymmetrisch ist und eine Winkelposition des Instruments vorgibt, wenn der Leuchtzeiger wie schon beschrieben auf eine Winkelmarkierung der Positionshilfe ausgerichtet wird. Die Ausrichtung des ersten Projektors relativ zu dem Instrument ist dann nicht mehr von einer Drehung des Instruments abhängig.

Auf vorteilhafte Weise kann aufgrund der gewonnenen Freiheitsgrade mittels einer veränderbaren Position des ersten Projektors die Positionierhilfe an gut sichtbarer Stelle vorgesehen werden und so das Ausrichten für den Nutzer vereinfacht werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Magnetresonanztomographen mit einem erfindungsgemäßen Instrument;
- Fig. 2: eine schematische Darstellungsform einer Ausführungsform eines erfindungsgemäßen Magnetresonanztomographen mit einem erfindungsgemäßen Instrument;
- Fig. 3: eine schematische Darstellungsform einer Ausführungsform eines erfindungsgemäßen Instruments;
- Fig. 4: eine schematische Darstellungsform einer Ausführungsform eines erfindungsgemäßen Magnetresonanztomographen mit einem erfindungsgemäßen Instrument;
- Fig. 5: eine schematische Darstellungsform einer Ausführungsform eines erfindungsgemäßen Instruments;
- Fig. 6: eine schematische Darstellungsform einer Ausführungsform eines erfindungsgemäßen Magnetresonanztomographen;
- Fig. 7: eine schematische Darstellungsform einer Ausführungsform eines erfindungsgemäßen Instruments;
- Fig. 8: einen schematischen Ablaufplan eines erfindungsgemäßen Verfahrens.

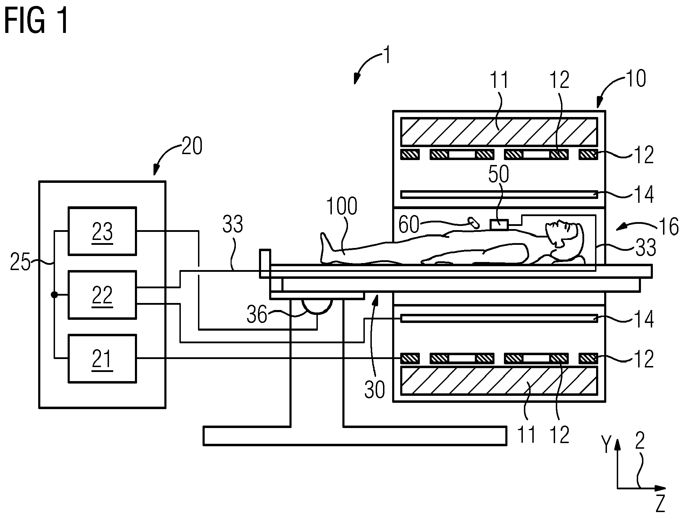

Fig. 1 zeigt eine schematische Darstellung einer Ausführungsform eines Magnetresonanztomographen 1 zum Ausführen des erfindungsgemäßen Verfahrens.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Magnetfeldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 zeitlich und räumlich variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungssignale können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Ein medizinisches Instrument 60, beispielsweise eine Biopsienadel, ist an einem Patienten 100 in einem Patiententunnel 16 angeordnet.

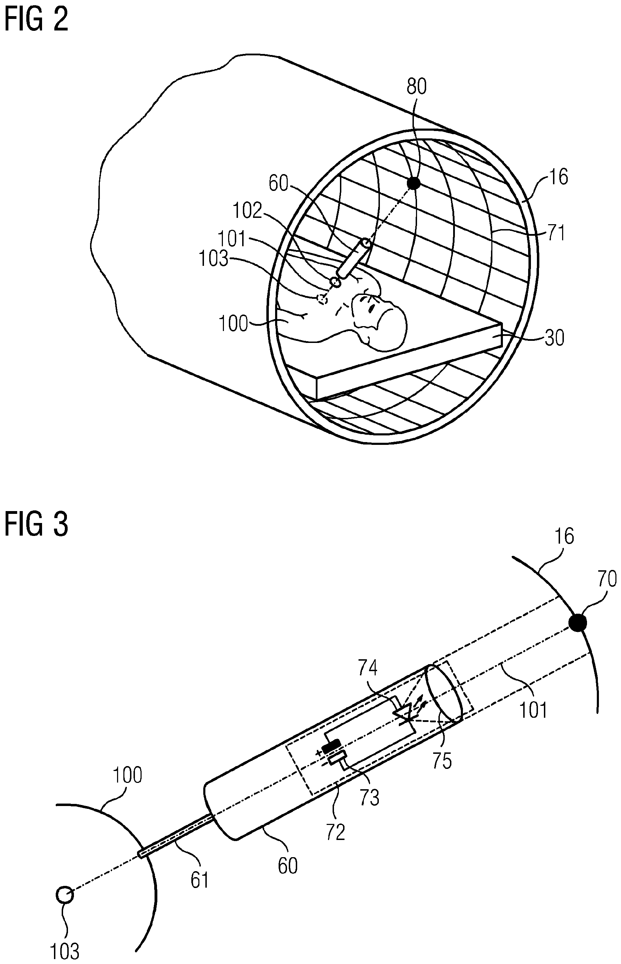

Fig. 2 zeigt einen Ausschnitt einer beispielhaften Ausführungsform aus Fig. 1 im Detail.

Im Patienten ist ein Zielpunkt 103 vorbestimmt, beispielsweise ein Knoten, der in einer Abbildung des Patienten 100 mit seinen Koordinaten identifiziert wurde. Ein medizinisches Instrument 60, beispielsweise eine Biopsienadel, mit der eine Probe aus dem Zielobjekt am Zielpunkt 103 entnommen werden soll, ist an einem Eintrittspunkt an der Oberfläche des Patienten angesetzt, der durch eine Markierung 102 ausgezeichnet ist. Die Markierung ist vorzugsweise auch in einem Magnetresonanzbild sichtbar. Denkbar ist aber auch eine Markierung 102 allein durch den Eindruck des Instruments 60 in den Patienten, die ebenfalls im Magnetresonanzbild sichtbar wird. Eine Trajektorie 101, eine Gerade, verbindet den Eintrittspunkt und den Zielpunkt 103, wobei die Trajektorie vorzugsweise so ausgewählt ist, dass sie kein wichtiges Organ, Blutgefäß, Nerv oder anderes zu schützendes Gewebe trifft.

Die vorbestimmte Trajektorie 101 weist ein auf einer von dem Patienten 100 abgewandten Seite einen Schnittpunkt mit dem Patiententunnel 16 bzw. dessen Innenwand auf. Dieser Schnittpunkt lässt sich bei bekannter Relativposition des Patienten 100 zu dem Magnetresonanztomographen 1 mit den Methoden der linearen Algebra berechnen. Eine Möglichkeit zum Bestimmen von Trajektorie 101 und Schnittpunkt bietet eine Magnetresonanzaufnahme des Patienten 100, in der das Zielobjekt 103 und der geplante Eintrittspunkt sichtbar ist. Der z.B. von der Steuerung 23 errechnete Schnittpunkt kann an einen Nutzer in Form von Koordinaten für ein an der Innenwand sichtbar angebrachtes Koordinatensystem 71 ausgegeben werden. Auf diese Weise kann der Nutzer die vorbestimmte Position 80 an der Innenwand des Patiententunnels 16 identifizieren, an der die Trajektorie 101 die Innenwand schneidet. Der Nutzer kann das Instrument 60 der Fig. 2 parallel zu der Trajektorie 101 und damit auf das Zielobjekt 103 ausrichten, wenn dieses wie näher in Fig. 3 dargestellt einen Lichtpunkt bzw. einen Leuchtzeiger 70 projiziert und er diesen Leuchtzeiger 70 in Deckung mit der vorbestimmten Position 80 bringt. Dabei ist es auch denkbar, dass das Koordinatensystem 71 mit einem zweiten Projektor 90 an die Innenwand des Patiententunnels 16 projiziert wird.

In Fig. 3 ist eine erfindungsgemäße Ausführungsform des medizinischen Instruments schematisch dargestellt. Das medizinische Instrument 60 weist eine Symmetrieachse auf, die hier durch die Biopsienadel 61 definiert ist. Die Symmetrie zu der Symmetrieachse spiegelt eine Symmetrie des medizinischen Instruments 60 bzw. der damit durchzuführenden Anwendung wider. Im Fall der Biopsienadel ist diese bei der Anwendung ohne weiter Konsequenzen um eine Achse durch die Längserstreckung der Hohlnadel 61 rotierbar. Demzufolge ist die Symmetrieachse des medizinischen Instruments 60 in Fig. 2 eine Rotationsachse durch die Längserstreckung der Hohlnadel 61. Wird diese in Deckung mit der vorbestimmten Trajektorie 101 gebracht, dann ist die Ausrichtung des medizinischen Instruments 60 korrekt auf das Zielobjekt 103 ausgerichtet.

Das medizinische Instrument 60 der Fig. 3 weist zu diesem Zweck einen ersten Projektor 72 auf. Der erste Projektor 72 weist eine Energiequelle auf, beispielsweise eine Batterie 73. Denkbar ist als Energiequelle aber auch eine Stromversorgungsleitung. Die Energiequelle versorgt eine Lichtquelle, vorzugsweise einen Halbleiterlaser 74. Denkbar sind aber auch andere Lichtquellen wie Lampen oder eine OLED-Matrix. Das erzeugte Licht wird von einer Optik 75 in einen Lichtstrahl gebündelt, der parallel zu der Symmetrieachse des medizinischen Instruments 60 in eine Richtung entgegengesetzt zu dem Patienten 100 abgestrahlt wird. Wenn der Lichtstrahl auf eine Oberfläche trifft, beispielsweise die Innenwand des Patiententunnels 16, so erzeugt er dort einen Lichtzeiger 70 in Form eines Flecks oder Punktes oder wie nachfolgend ausgeführt eines komplexeren Musters.

Ist das medizinische Instrument 60 mit der Spitze bzw. der Hohlnadel 61 am Eintrittspunkt angeordnet und richtet der Nutzer den Leuchtzeiger 70 auf die vorbestimmte Position 80 aus, so ist durch diese zwei Fixpunkte eine Gerade definiert, die der vorbestimmten Trajektorie 101 entspricht und eine erfindungsgemäße Ausrichtung des medizinischen Instruments 60 sicherstellt.

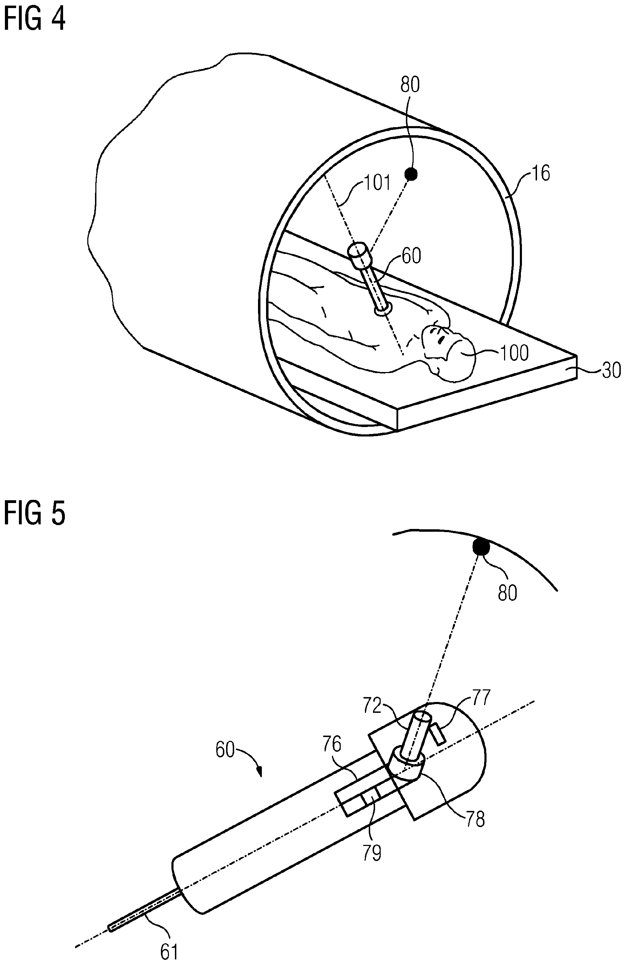

In Fig. 4 ist eine weitere mögliche Ausführungsform des erfindungsgemäßen medizinischen Instruments 60 und des erfindungsgemäßen Magnetresonanztomographen 1 dargestellt. Gleiche Referenzzeichen bezeichnen gleiche Gegenstände, insbesondere ist die Trajektorie 101 weiterhin als die Gerade durch Eintrittspunkt und Zielobjekt 103 definiert. Die in Fig. 4 dargestellt Ausführungsform unterscheidet sich im Kern dadurch, dass die Trajektorie 101 nicht mehr mit der Richtung der Projektion des Leuchtzeigers 70 übereinstimmt. Dadurch ist es möglich, als vorbestimmte Position 80 nicht mehr immer wechselnde Koordinaten auf der Innenseite des Patiententunnels 16 auszuwählen, sondern nur eine oder wenige vorbestimmte Positionen 80 auszuzeichnen, die dann beispielsweise durch eine feste Markierung wie eine Erhebung oder Vertiefung oder einen Reflexpunkt ausgezeichnet sind. Es muss jedoch jeweils die Ausrichtung des ersten Projektors 72 relativ zu dem medizinischen Gerät 60 geändert werden, wie im Detail zu Fig. 5 dargestellt wird.

In Fig. 5 ist der erste Projektor 72 nicht mehr parallel zu der Symmetrieachse des medizinischen Instruments 60 ausgerichtet, sondern mittels eines ersten Aktuators 77 und eines optionalen zweiten Aktuators 78 relativ zu der Symmetrieachse veränderbar. Beispielsweise kann eine Projektorsteuerung 76 in dem medizinischen Instrument vorzugsweise drahtlos Einstellanweisung von der Steuerung 23 des Magnetresonanztomographen 1 erhalten. Die Steueranweisungen können eine Winkelausrichtung zu der Symmetrieachse mittels des ersten Aktuators 77 und/oder eine Winkelausrichtung bezüglich einer Rotation um die Symmetrieachse mittels des zweiten Aktuators 78 vorgeben. Der erste Aktuator und der zweite Aktuator können beispielsweise Motoren, vorzugsweise piezoelektrische Aktuatoren sein, die eine lineare Bewegung oder eine Rotation ausführen, ohne ein störendes Magnetfeld zu benötigen.

Die einzustellenden Winkel sind dabei von einer Rotation des Instruments 60 um die Symmetrieachse relativ zu dem Magnetresonanztomographen oder auch der Schwerkraftrichtung abhängig. Diese Rotation kann mittels eines Sensors 79 von der Projektorsteuerung 76 erfasst und der Steuerung 23 zum Ermitteln der Winkel für den ersten Aktuator 77 und den zweiten Aktuator 78 übertragen werden. Der Sensor 79 kann beispielsweise ein Gravitationssensor in MEMS-Technologie sein oder ein mehrdimensionaler Hall-Sensor.

Denkbar wäre es aber auch, den ersten Projektor 72 frei drehbar um die Rotationsachse auszulegen und die Gewichtsverteilung so auszuführen, dass der erste Projektor 72 unter Einwirkung der Schwerkraft eine Vorzugsrichtung aufweist, vorzugsweise entgegen der Schwerkraft nach oben gerichtet.

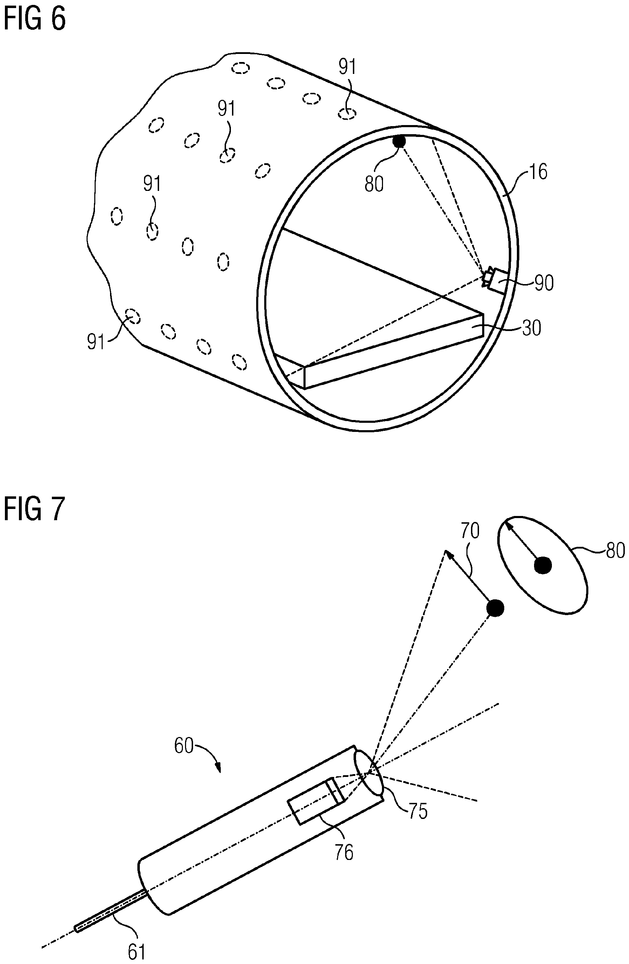

In Fig. 6 sind zwei Möglichkeiten dargestellt, wie in einem erfindungsgemäßen Magnetresonanztomographen 1 die vorbestimmte Position 80 ausgezeichnet werden kann.

Denkbar ist ein zweiter Projektor 90, der in oder an dem Patiententunnel 16 angebracht ist und in dessen Inneres projiziert. Der zweite Projektor 90 kann dabei beispielsweise ein Bild mit einer veränderlichen Position und/oder Ausrichtung als vorbestimmte Position 80 für den Leuchtzeigers 70 projizieren. Das Bild kann beispielsweise mit einer OLED-, LCD- oder DLP-Matrix erzeugt werden. Denkbar ist aber auch eine variable Ausrichtung des zweiten Projektors 90 mit Aktuatoren, wie sie in Fig. 5 zu dem ersten Projektor 72 dargelegt wurde.

Eine variable Auszeichnung der vorbestimmten Position 80 kann aber beispielsweise auch durch eine schaltbare Matrix an Leuchtpunkten 91 an der Innenwand des Patiententunnels 16 erzeugt werden, die z.B. durch LEDs erzeugt werden und optional durch Lichtleiter zu der Innenwand des Patiententunnels 16 übertragen werden.

In Fig. 7 ist eine weitere Ausführungsform eines erfindungsgemäßen medizinischen Instruments 60 schematisch dargestellt. Die dargestellt Ausführungsform stellt eine Alternative zu der in Fig. 5 dargestellten Variante dar. Anstatt den ersten Projektor 72 mittels eines ersten Aktuators 77 und/oder zweiten Aktuators 78 unterschiedlich auszurichten, wird das projizierte Bild verändert. Dabei weist der erste Projektor 72 einen Projektionswinkel auf, der vorzugsweise größer als 20 Grad, 40 Grad oder 60 Grad ist. Dadurch kann der Leuchtzeiger 70 unter vielen unterschiedlichen Ausrichtungen der Trajektorie 101 in Deckung mit der vorbestimmten Position 80 gebracht werden. Dabei ist es denkbar, dass der Leuchtzeiger 70 eine Position und eine Richtung auszeichnet, beispielsweise durch den Ursprungspunkt und die Ausrichtung des Pfeils in Fig. 7. Indem dieser Leuchtzeiger 70 in Deckung mit einer vorbestimmten Position 80, ebenfalls ausgezeichnet durch eine Orts- und eine Richtungsmarkierung, gebracht wird, kann auch eine definierte Position des medizinischen Instruments 60 für eine Rotation um die Symmetrieachse erzielt werden.

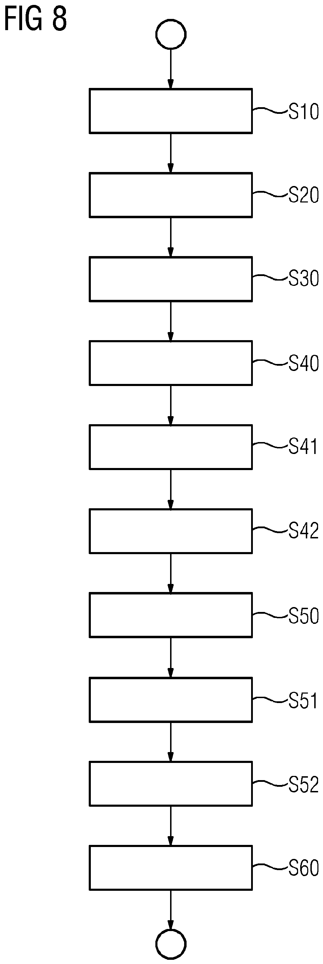

Fig. 8 zeigt einen schematischen Ablaufplan eines erfindungsgemäßen Verfahrens.

In einem Schritt S30 wird der Patienten 100 in dem Magnetresonanztomographen 1 positioniert, vorzugsweise mit dem Patiententisch 30. An dem Patienten 100 soll eine vorzugsweise minimalinvasive Untersuchung oder ein Eingriff mittels eines medizinischen Instruments 60 an einem Organ oder Gewebe vorgenommen werden, das im Folgenden als Zielobjekt 103 bezeichnet wird. Wie dieser Eintrittspunkt bestimmt werden kann, wird nachfolgend noch erläutert. Das Positionieren erfolgt dabei so, dass der Eintrittspunkt und das Zielobjekt 103 in einem Erfassungsbereich des Magnetresonanztomographen 1 befinden, der auch als Field of View (FoV) bezeichnet wird. Die korrekte Position kann beispielsweise anhand von Markierungen 102 am Patienten 100 und Magnetresonanztomographen 1 oder einer schnellen Bilderfassung mit dem Magnetresonanztomographen 1 verifiziert werden. Am Ende des Positionierens befindet sich der Patient 100 in einer bekannten Relativposition und Ausrichtung relativ zu dem Magnetresonanztomographen 1.

In einem anderen Schritt S40 des erfindungsgemäßen Verfahrens wird eine vorbestimmte Ausrichtung des ersten Projektors 72 relativ zu dem medizinischen Instrument 60 und/oder Position der Positionierhilfe an der Wand des Patiententunnels 16 ermittelt, in der der Leuchtzeiger 70 des ersten Projektors 72 in Deckung mit der Positionierhilfe kommt, wenn das medizinische Instrument parallel zu einer Trajektorie 101 durch den Eintrittspunkt und den Zielpunkt ausgerichtet ist.

Der Schritt S40 unterscheidet sich dabei für die Fälle, dass der erste Projektor 72 wie in Fig. 3 parallel zur Symmetrieachse des medizinischen Instruments ausgerichtet ist oder wie in Fig. 5 oder Fig. 7 selbst zur Symmetrieachse ausrichtbar ist.

Für die Ausführungsform der Fig. 3 mit Symmetrieachse parallel zur Trajektorie 101 wird in einem Schritt S41 ein Schnittpunkt der Trajektorie durch vorbestimmten Eintrittspunkt und Zielobjekt 103 mit der Innenwand des Patiententunnels 16 auf einer von dem Patienten 100 abgewandten Seite des Instruments 60 bestimmt. Dies kann beispielsweise durch die Lösung eines Gleichungssystems erfolgen, in dem die Geradengleichung der Trajektorie 101 mit einer Hüllkurve der Innenwand gleichgesetzt wird.

In den Ausführungsformen der Fig. 5 und 7 ist eine Ausrichtung des ersten Projektors 72 in einem Winkel relativ zu der Symmetrieachse des medizinischen Instruments 60 und einem Rotationswinkel um die Symmetrieachse einstellbar. Die vorbestimmte Position 80 kann dabei unveränderlich an der Innenwand des Patiententunnels 16 angeordnet sein und graphisch oder durch eine Struktur markiert sein. In diesem Fall wird die Ausrichtung des ersten Projektors 72 in einem Schritt S42 so bestimmt, dass der Leuchtzeiger 70 des ersten Projektors auf die vorbestimmte Position 80 fällt, wenn das medizinische Instrument 60 anwendungsgemäß parallel zu der Trajektorie 101 ausgerichtet ist. Dies kann durch Lösen eines Gleichungssystems erfolgen, bei dem die Trajektorie 101 mit einer Drehmatrix multipliziert wird, die als Variable die beiden Winkel der Ausrichtung des ersten Projektors 72 enthält, und mit der vorbestimmten Position 80 gleichgesetzt wird. Durch Auflösen nach den beiden Winkeln wird die Ausrichtung des ersten Projektors 72 bestimmt, beispielsweise durch die Steuerung 23 des Magnetresonanztomographen 1. Dabei ist es auch denkbar, dass beim Ermitteln die Projektorsteuerung 76 mittels eines Sensors 79 eine Lage des medizinischen Instruments 60 relativ zu dem Magnetresonanztomographen 1 ermittelt und die Ausrichtung in Abhängigkeit von dieser Lage ermittelt wird. Beispielsweise kann diese Lage als Offset in den Rotationswinkel eingehen.

In einem Schritt S50 wird das Ergebnis des Ermittelns in Schritt S40 ausgegeben.

Für eine Ausführungsform des medizinischen Instruments 60 mit fester paralleler Ausrichtung des ersten Projektors 72 und des davon erzeugten Leuchtzeigers 70 zu der Symmetrieachse kann die Koordinate für die vorbestimmte Position 80 in einem Schritt S51 auf einer Ausgabe des Magnetresonanztomographen 1 ausgegeben werden. Denkbar ist auch, dass ein zweiter Projektor 90 die vorbestimmte Position 90 anzeigt, indem der zweite Projektor 90 von Aktuatoren auf die vorbestimmte Position 80 von der Steuerung 23 ausgerichtet wird. Möglich ist auch, dass der zweite Projektor 90 fest ausgerichtet ist, aber ein veränderliches Bild mit einer einstellbaren Position einer projizierten Markierung für die vorbestimmte Position 80 ausgibt, das beispielsweise von der Steuerung 23 eingestellt wird.

Für eine Ausführungsform, bei der der erste Projektor 72 oder zumindest der von ihm projizierte Leuchtzeiger 80 in der Ausrichtung relativ zu dem medizinischen Instrument 60 veränderlich ist, erfolgt das Ausgeben in einem Schritt S52 beispielsweise, indem erster Aktuator 77 und zweiter Aktuator 78 entsprechend durch eine Einstellungsanweisung der Steuerung 23 an die Projektorsteuerung 76 ausgerichtet werden.

Denkbar ist in einer Ausführungsform mit einem fixierten ersten Projektor 72 der ein Bild projiziert, dass die Projektorsteuerung 76 das Bild so einstellt, dass der Leuchtzeiger 70 in der ermittelten Ausrichtung relativ zu dem medizinischen Instrument 60 projiziert wird.

Anschließend wird der Leuchtzeiger 70 von dem ersten Projektor 72 projiziert.

In einem anderen Schritt S60 wird das medizinische Instrument 60 an dem vorbestimmten Eintrittspunkt positioniert. Dies erfolgt vorzugsweise durch einen Nutzer und kann auch bereits zu einem früheren Zeitpunkt des Verfahrens erfolgen.

In einem Schritt S60 wird schließlich das medizinische Instrument derart ausgerichtet, dass der Leuchtzeiger 70 mit der Positionierhilfe in Deckung gelangt. Vorzugsweise erfolgt dies durch den Nutzer, der das medizinische Instrument 60 solange neigt, schwenkt und/oder rotiert, bis der Lichtzeiger 70 mit der vorbestimmten Position 80 in Deckung kommt, wobei die Spitze des medizinischen Instruments 60 weiterhin am Eintrittspunkt verbleibt. In einer Ausführungsform, in der der Lichtzeiger 70 und die Markierung für die vorbestimmte Position 80 eine Richtungsmarkierung zur Anzeige einer Richtungsinformation aufweist, kann dabei das Ausrichten des medizinischen Instruments 60 auch das Rotieren des medizinischen Instruments 60 um die Symmetrieachse umfassen, bis die Richtungsmarkierungen in Deckung kommen.

In einer Ausführungsform des erfindungsgemäßen Verfahrens weist das Verfahren weiterhin den Schritt S10 auf, eine Abbildung des Patienten 100 auf der Bedienoberfläche der Planungssteuerung auszugeben. Die Planungssteuerung kann dabei identisch mit der Steuerung 23 des Magnetresonanztomographen 1 sein. Die Abbildung enthält dabei zumindest das Zielobjekt 103 und einen Bereich um einen möglichen Eintrittspunkt. Vorzugsweise handelt es sich bei der Abbildung um eine Magnetresonanztomographie mit dem Magnetresonanztomographen 1. Es ist aber auch denkbar, dass die Abbildung mit einer anderen Modalität erfasst wurde und auf einem Datenträger oder über eine Datennetzwerkverbindung zur Verfügung gestellt wird.

In einem weiteren Schritt S20 wird ein Eintrittspunkt auf der Patientenoberfläche und ein Zielpunkt in dem Zielgebiet 103 in dem Patienten 100 festgelegt. Der Zielpunkt ist üblicherweise durch ein in der Abbildung erkennbares Organ oder Gewebe definiert, beispielsweise einem Knoten oder ein ganzes Organ. Der Eintrittspunkt kann bereits in der Abbildung durch einen von der Modalität erfassten Marker gekennzeichnet sein. Es ist aber auch denkbar, dass dieser erst anhand der Abbildung festgelegt wird, beispielsweise unter der Anforderung, auf dem Weg vom Eintrittspunkt zum Zielgebiet kein sensibles Gewebe zu verletzen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Medizinisches Instrument zur Anwendung an oder in einem Patienten (100), wobei das Instrument (60) einen ersten Projektor (72) zur Projektion eines Leuchtzeigers (70) in einer vorbestimmten Ausrichtung relativ zu dem medizinischen Instrument (60) aufweist, **dadurch gekennzeichnet, dass** die vorbestimmte Ausrichtung bei einer anwendungsgemäßen Positionierung des medizinischen Instruments (60) am Patienten (100) von dem Patienten (100) weg zeigt.

2. Medizinisches Instrument nach Anspruch 1, wobei das medizinische Instrument (60) eine Symmetrieachse aufweist und die vorbestimmte Richtung parallel zu der Symmetrieachse ausgerichtet ist.

3. Medizinisches Instrument nach einem der vorhergehenden Ansprüche, wobei das medizinische Instrument (60) einen Steuereingang aufweist und ausgelegt ist, die vorbestimmte Ausrichtung des ersten Projektors (72) in Abhängigkeit von einer Anweisung über den Steuereingang auf vorbestimmte Weise einzustellen.

4. Medizinisches Instrument nach Anspruch 3, wobei das medizinische Instrument (60) einen Sensor (79) aufweist, der ausgelegt ist, eine Ausrichtung des medizinischen Instruments (60) relativ zu dem Magnetresonanztomographen (1) zu erfassen.

5. Magnetresonanztomograph, wobei der Magnetresonanztomograph (1) eine Positionierhilfe aufweist, die ausgelegt ist, eine vorbestimmte Position (80) an einer Innenwand eines Patiententunnels (16) für einen Anwender sichtbar auszuzeichnen.

6. Magnetresonanztomograph nach Anspruch 5, wobei die Positionierhilfe ein sichtbar auf der Innenseite des Patiententunnels (16) angebrachte Markierung aufweist.

7. Magnetresonanztomograph nach Anspruch 5, wobei der Magnetresonanztomograph (1) einen zweiten Projektor (90) aufweist, wobei der zweite Projektor (90) ausgelegt ist, eine vorbestimmte Position (80) an der Innenwand des Patiententunnels (16) mittels einer Lichtprojektion sichtbar auszuzeichnen.

8. System aus einem medizinischen Instrument (60) nach einem der Ansprüche 1 bis 4 und einem Magnetresonanztomographen (1) nach Anspruch 5 bis 7 und einer Planungssteuerung mit einer Bedienoberfläche zur Planung eines Eingriffs mit dem medizinischen Instrument (60).

9. Verfahren zum Positionieren eines medizinischen Instruments (60) an einem Patienten (100) mit einem System nach Anspruch 8, wobei das Verfahren die Schritte aufweist:
(S30) Positionieren des Patienten (100) in dem Magnetresonanztomographen (1) derart, dass sich ein vorbestimmter Eintrittspunkt für das medizinische Instrument (60) an der Oberfläche des Patienten (100) und ein vorbestimmter Zielpunkt des Eingriffs in dem Patienten (100) in einem Erfassungsbereich des Magnetresonanztomographen (1) befinden;
(S40) Ermitteln einer vorbestimmten Ausrichtung des ersten Projektors (72) relativ zu dem medizinischen Instrument (60) und/oder Position der Positionierhilfe an der Wand des Patiententunnels (16), in der der Leuchtzeiger (70) des ersten Projektors (72) in Deckung mit der Positionierhilfe kommt,
wenn das medizinische Instrument (60) parallel zu einer Trajektorie (101) durch den Eintrittspunkt und den Zielpunkt ausgerichtet ist;
(S50) Ausrichten des ersten Projektors (72) und/oder Ausgeben der Positionierhilfe sowie projizieren des Leuchtzeigers (70).

10. Verfahren nach Anspruch 9, wobei das Verfahren weiterhin den Schritt (S60) aufweist, das medizinische Instrument (60) derart auszurichten, dass der Leuchtzeiger (70) mit der Positionierhilfe in Deckung gelangt.

11. Verfahren nach Anspruch 9 oder 10, wobei das Verfahren weiterhin die Schritte aufweist:
(S10) Bereitstellen einer Abbildung eines Patienten (100) auf der Bedienoberfläche der Planungssteuerung;
(S20) Festlegen eines Eintrittspunktes auf der Patientenoberfläche und eines Zielpunktes in dem Patienten (100).

12. Verfahren nach einem der Ansprüche 9 bis 11, wobei die Ausrichtung des ersten Projektors (72) zu dem medizinischen Instrument (60) fest und vorzugsweise parallel zu einer Symmetrieachse des medizinischen Instruments (60) ist, in Schritt (S40) Ermitteln der Ausrichtung in einem Schritt (S41) ein Schnittpunkt des Leuchtzeigers (60) mit der Innenseite des ermittelt wird und in Schritt (S50) Ausrichten der Schnittpunkt ausgegeben wird.

13. Verfahren nach Anspruch 12, wobei das Ausgeben des Schnittpunkts das Ausgeben einer Koordinate für ein Koordinatensystem auf der Innenseite des Patiententunnels (16) an einen Nutzer des medizinischen Instruments (60) ist.

14. Verfahren nach Anspruch 13 mit einem Magnetresonanztomographen (1) nach Anspruch 7, wobei das Ausgeben des Schnittpunktes ein Schritt (S51) Markieren mittels des zweiten Projektors (90) an der Innenseite des Patiententunnels (16) ist.

15. Verfahren nach einem der Ansprüche 9 bis 11, wobei die Ausrichtung des ersten Projektors (72) zu dem medizinischen Instrument (60) von dem Magnetresonanztomographen (1) einstellbar ist, und in Schritt (S40) Ermitteln der Ausrichtung in einem Schritt (S42) eine Ausrichtung des ersten Projektors (72) relativ zu dem medizinischen Gerät (60) bestimmt wird, unter der der Lichtzeiger (70) bei Ausrichtung des medizinischen Instruments (60) parallel zu einer Trajektorie durch den Eintrittspunkt und den Zielpunkt auf die Positionierhilfe ausgerichtet ist und in dem Schritt des Ausgebens in einem Schritt (S52) der erste Projektor (72) gemäß der bestimmten Ausrichtung ausgerichtet wird.

16. Computerprogrammprodukt, welches ein Programm umfasst und direkt in einen Speicher einer programmierbaren Steuerung (23) eines Magnetresonanztomographen (1) nach Anspruch 5 bis 7 ladbar ist, mit Programmmitteln, um alle Schritte des Verfahrens nach einem der Ansprüche 9 bis 15 auszuführen, wenn das Programm in der Steuerung (23) des Magnetresonanztomographen (1) ausgeführt wird.

17. Elektronisch lesbarer Datenträger mit darauf gespeicherten elektronisch lesbaren Steuerinformationen, welche derart ausgestaltet sind, dass sie bei Verwendung des Datenträgers in einer Steuerung (23) eines Magnetresonanztomographen (1) nach einem der Ansprüche 5 bis 7 das Verfahren nach einem der Ansprüche 9 bis 15 durchführen.
